(19) Europäisches Patentamt
European Patent Office
Office européen des brevets

(11) Veröffentlichungsnummer: **0 303 186 B1**

(12) **EUROPÄISCHE PATENTSCHRIFT**

(45) Veröffentlichungstag der Patentschrift: **10.11.93**

(51) Int. Cl.5: **C07F 9/40**, C07F 9/38, C07F 9/32, C07F 9/30, C07F 9/53, A61K 31/66

(21) Anmeldenummer: **88112704.7**

(22) Anmeldetag: **04.08.88**

# (54) Arylphosphorderivate, ihre Herstellung und Verwendung.

(30) Priorität: **12.08.87 DE 3726806**

(43) Veröffentlichungstag der Anmeldung:
**15.02.89 Patentblatt 89/07**

(45) Bekanntmachung des Hinweises auf die Patenterteilung:
**10.11.93 Patentblatt 93/45**

(84) Benannte Vertragsstaaten:
**BE CH DE FR GB IT LI NL SE**

(56) Entgegenhaltungen:
**EP-A- 0 176 050**

(73) Patentinhaber: **BASF Aktiengesellschaft**
**Carl-Bosch-Strasse 38**
**D-67063 Ludwigshafen(DE)**

(72) Erfinder: **Wuest, Hans-Heiner, Dr.**
**Unteres Bieth 10**
**D-6915 Dossenheim(DE)**
Erfinder: **Janssen, Bernd, Dr.**
**Leuschnerstrasse 18a**
**D-6700 Ludwigshafen(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.6/3.3.1)

## Beschreibung

Die Erfindung betrifft neue Phosphonsäure-, Phosphinsäure- und Phosphinoxidderivate, Verfahren zu deren Herstellung sowie deren Verwendung bei der Bekämpfung von Krankheiten.

Aus den DE-OS 2 854 354 und DE-OS 3 202 118 ist bekannt, daß retinoidale Benzoesäurederivate pharmakologische Wirkungen bei der topischen und systemischen Therapie von Neoplasien und Dermatosen, z.B. Akne oder Psoriasis aufweisen. Nachteil dieser Verbindungen ist ihre geringe therapeutische Breite bezüglich der unter dem Begriff Hypervitaminose A zusammengefaßten Nebenwirkungen. EP 176 050 beschreibt retinoidale Vinylbenzylphosphorsäureester mit ähnlicher Wirksamkeit. Sie sind jedoch metabolisch instabil; sie werden am Pharmakophor verseift.

Es wurde nun gefunden, daß Arylphosphorderivate der allgemeinen Formel

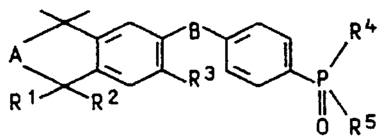

I,

in der

A einen gegebenenfalls mit einer Methylgruppe substituierten Ethylen- oder Methylenrest,

B eine $-CR^6=CH-$ oder $-C{\equiv}C-$Gruppe,

$R^1$ und $R^2$ Wasserstoff oder einen Methylrest,

$R^3$ Wasserstoff, einen $C_{1-4}$-Alkylrest, ein Halogenatom, vorzugsweise Fluor, oder eine Methoxygruppe und

$R^4$ und $R^5$ einen $C_{1-6}$-Alkylrest, einen Phenylrest, einen Rest $-OR^7$ oder einen Rest $-NR^8R^9$ bedeuten, wobei

$R^6$ für Wasserstoff oder einen $C_{1-4}$-Alkylrest,

$R^7$, $R^8$ und $R^9$ für Wasserstoff, einen gegebenenfalls durch eine Hydroxylgruppe substituierten $C_1$-$C_4$-Alkylrest oder eine Phenylgruppe stehen,

wobei zwei an Phosphor gebundene Heteroatome durch eine $C_{2-3}$-Alkylengruppe verbunden sein können, oder $R^8$ und $R^9$ zusammen mit dem Stickstoffatom, an das sie gebunden sind, für einen heterocyclischen Rest, vorzugsweise Morpholin, Pyrrolidin oder Piperidin, stehen,

sowie deren physiologisch verträglichen Salze ein verbessertes Wirkprofil, vor allem hinsichtlich der Nebenwirkungen, besitzen.

Die erfindungsgemäßen Verbindungen kann man herstellen, indem man

a) einen Aldehyd oder ein Keton der Formel II

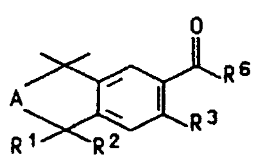

II,

in der $R^1$, $R^2$, $R^3$, $R^6$ und A die oben angegebenen Bedeutungen haben, in einer Wittig-Horner-Reaktion mit einem Phosphonat der Formel III

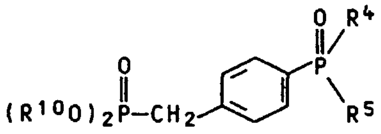

III,

in der $R^4$ und $R^5$ die oben angegebene Bedeutung haben und $R^{10}$ eine $C_{1-4}$-Alkylgruppe bedeutet, umsetzt oder

b) eine Phosphoniumverbindung der Formel IV

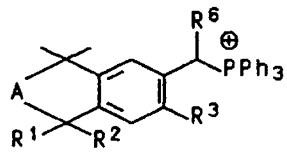 $X^{\ominus}$ IV,

in der $R^1$, $R^2$, $R^3$, $R^6$ und A die oben angegebenen Bedeutungen haben und X ein anorganisches Anion ist, in einer Wittig-Reaktion mit einem Aldehyd der Formel V

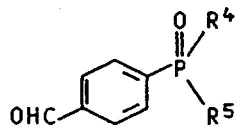 V,

in der $R^4$ und $R^5$ die oben angegebene Bedeutung haben, umsetzt oder
c) indem man ein Arylhalogenid der Formel VI

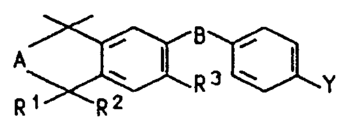 VI,

in der $R^1$, $R^2$, $R^3$, A und B die oben angegebenen Bedeutungen haben und Y ein Halogenatom bedeutet, zweckmäßigerweise in Gegenwart eines Übergangsmetallkatalysators, mit einer Phosphorverbindung der Formel VII

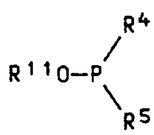 VII,

worin $R^4$ und $R^5$ die oben angegebene Bedeutung haben und $R^{11}$ einen $C_{1-6}$-Alkylrest bedeutet, umsetzt oder
d) ein Arylacetylen der Formel VIII

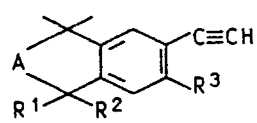 VIII,

worin $R^1$, $R^2$, $R^3$ und A die oben angegebenen Bedeutungen haben, mit einem Arylhalogenid der Formel IX

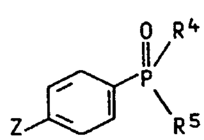 IX

worin $R^4$ und $R^5$ die obene angegebene Bedeutung haben und Y für ein Halogenatom steht, zweckmäßig

in Gegenwart eines Übergangsmetallkatalysators umsetzt und gegebenenfalls
e) die nach den Verfahren a) - c) erhaltenen Verbindungen nach Standardmethoden in weitere Verbindungen der Formel I umwandelt.

Die Halogenatome Y gemäß c) und Z gemäß d) sind vorzugsweise Brom oder Jod.

Die Umsetzung nach Wittig-Horner und Wittig gemäß a) und b) verläuft bei einer Temperatur von bis zu 100 °C, zweckmäßig bei 20 bis 50 °C. Die Umsetzung kann bei atmosphärischem Druck oder in einem geschlossenen Gefäß unter erhöhtem Druck durchgeführt werden.

Man kann diese Umsetzung in Gegenwart eines Verdünnungs- oder Losungsmittels, beispielsweise eines niederen gesättigten Dialkylethers, Dialkylglykolethers oder cyclischen Ethers, wie Diethylether, Ethyl-tert.-butylether, 1,2-Dimethoxyethan, Tetrahydrofuran oder Dioxan, eines aromatischen Kohlenwasserstoffs, wie Benzol, Toluol oder Xylol, oder eines gesättigten aliphatischen Kohlenwasserstoffs, wie Hexan, Heptan oder Isooctan, eines Dialkylformamids oder -sulfoxids, wie Dimethylformamid oder Dimethylsulfoxid, oder in Mischungen der genannten Lösungsmittel durchführen. Bevorzugt verwendet man cyclische Ether wie Tetrahydrofuran sowie insbesondere Dimethylformamid und Dimethylsulfoxid oder deren Mischungen, wobei die Umsetzung im allgemeinen bei einer Temperatur bis zu 30 °C abläuft.

Die Umsetzungen nach Wittig und Wittig-Horner werden in Gegenwart eines Deprotonierungsmittels für das Phosphonat II bzw. das Phosphoniumsalz IV vorgenommen. Geeignet sind Alkalimetallhydride und Alkalimetallamide, insbesondere des Natriums und Kaliums, die Natrium-und Kaliumsalze von Dimethylsulfoxid, Alkyllithiumverbindungen wie n-Butyllithium oder Alkalimetallalkoholate, vorzugsweise Natriumethanolat, Natriummethanolat oder Kalium-tert.-butanolat.

Die Wittig- oder Wittig-Horner-Reaktion liefert üblicherweise Gemische der sterisch isomeren (E/Z)-Olefine.

E/Z-Isomerengemische mit überwiegendem Z-Anteil werden unter Lichteinwirkung und/oder unter Zusatz katalytischer Mengen Jod an der olefinischen Doppelbindung zu Gemischen mit höherem E-Anteil isomerisiert. Aus den erhaltenen (E/Z)-Isomerengemischen mit jetzt günstigerem (E)-Gehalt werden vorteilhaft reine (E)-Verbindung der Formel I, vorzugsweise durch Kristallisation oder eine chromatographische Methode wie die Säulen- oder präparative HPL-Chromatographie erhalten.

Die Umsetzung gemäß c) wird, insbesondere wenn Y in Formel VI Chlor oder Brom bedeutet, aber auch wenn Y Jod bedeutet, zweckmäßigerweise durch einen Übergangsmetallkatalysator katalysiert, wie z.B. Kupferpulver, Salze oder Komplexe des Nickels und Palladiums, vorzugsweise Nickel(II)-chlorid oder Nickel-(II)-bromid. Die Umsetzung wird bei Temperaturen zwischen 100 und 200 °C durchgeführt, vorzugsweise zwischen 120 und 160 °C.

Man kann unter Zusatz von Lösungsmittel arbeiten, z.B. aromatischen Kohlenwasserstoffen wie Toluol oder Xylol.

Bei Reaktion d) werden aus Verbindungen der Formel VIII in üblicher Weise in situ die entsprechenden Kupferacetylide hergestellt, die mit den Arylhalogeniden IX weiter zu Verbindungen der Formel I umgesetzt werden. Alternativ kann die Kupplungsreaktion, direkt von Acetylenen VIII ausgehend, durch Komplexe des Palladiums und Nickels, z.B. mit Triphenylphosphin, katalysiert werden. In allen Fällen ist die Gegenwart einer Base zweckmäßig, beispielsweise einer organischen Stickstoffbase, wie Triethylamin oder Pyridin, oder eines Alkalimetallalkoholats, wie Natriummethanolat oder Natriumphenolat. Gegebenenfalls wird in einem Lösungsmittel gearbeitet, vorzugsweise in Dimethylformamid oder Tetrahydrofuran. Die Umsetzung verläuft bei einer Temperatur von 40 bs 150 °C, zweckmäßigerweise bei etwa 50 °C (Aryliodide) oder etwa 100 °C (Arylbromide).

zu e)

Durch Hydrolyse der Phosphonsäurediester und Phosphinsäureester der Formel I können in an sich bekannter Weise je nach Wahl der Hydrolysebedingungen die Phosphon- und Phosphinsäuren oder Phosphonsäuremonoester hergestellt werden. Die Verseifung der Phosphonsäurediester mit wäßrigen Hydroxiden der Alkali- und Erdalkalimetalle - bevorzugt sind Natrium- und Kaliumhydroxid - führt allgemein zu den entsprechenden Phosphonsäuremonoestern. Die vollständige Hydrolyse gelingt durch Reaktion der Phosphonsäurediester mit Trialkylhalogensilanen, vorzugsweise Trimethylbrom- und Trimethyliodsilan, die vorteilhafterweise in situ aus Trimethylchlorsilan und einem Alkalimetallbromid bzw. -iodid hergestellt werden, und anschließender Behandlung mit Wasser oder verdünnten Mineralsäuren, z.B. Salzsäure oder Schwefelsäure.

Aus den so erhaltenen Säuren lassen sich weitere erfindungsgemäße Verbindungen nach bekannten Verfahrensweisen herstellen. So kann man eine Phosphonsäure der Formel I beispielsweise mit Phosphorpentachlorid in das Phosphonsäuredichlorid überführen, welches mit Alkoholen oder Aminen zu den

entsprechenden Estern bzw. Amiden umgesetzt wird.

Aus Phosphonsäureestern, Phosphinsäureestern, Phosphonsäurechloriden oder Phosphinsäurechloriden können durch Umsetzung mit metallorganischen Reagentien, z.B. Grignardverbindungen, entsprechende Phosphanoxidderivate erhalten werden.

Die erfindungsgemäßen Verbindungen und ihre physiologisch verträglichen Salze können aufgrund ihrer pharmakologischen Eigenschaften bei der topischen und systemischen Therapie und auch Prophylaxe von Praekanzerosen und Karzinomen der Haut, der Schleimhäute und inneren Organe sowie bei der topischen und systemischen Therapie von Akne, Psoriasis und anderer mit pathologisch veränderter Verhornung einhergehenden dermatologischen Erkrankungen sowie zur Behandlung von rheumatischen Erkrankungen, insbesondere solchen entzündlicher oder degenerativer Art, die Gelenke, Muskeln, Sehnen und andere Teile des Bewegungsapparates befallen, verwendet werden. Ein bevorzugtes Indikationsgebiet ist neben der Therapie von dermatologischen Erkrankungen die prophylaktische Behandlung von Praekanzerosen und Tumoren.

Die pharmakologischen Wirkungen können beispielsweise in den nachfolgenden Testmodellen aufgezeigt werden: Die erfindungsgemäßen Verbindungen heben an Hamstertrachealgewebe in vitro die nach Vitamin-A-Mangel einsetzende Keratinisierung auf. Die Keratinisierung gehört zur Frühphase der Carcinogenese, die in einer ähnlichen Technik in vivo nach der Initiation durch chemische Verbindungen, durch energetische Strahlung oder nach viraler Zelltransformation durch die erfindungsgemäßen Verbindungen der Formel I inhibiert wird. Diese Methodik kann aus Cancer Res. 36, 964 - 972 (1972) oder aus Nature 250, 64 - 66 (1974) und Nature 253, 47 - 50 (1975) entnommen werden.

Darüber hinaus werden durch die erfindungsgemäßen Verbindungen die Proliferationsraten bestimmter maligne veränderter Zellen inhibiert. Diese Methodik kann aus J. Natl. Cancer Inst. 60, 1035 - 1041 (1978), Experimental Cell Research 117, 15 - 22 (1978) und Proc. Natl. Acad. Sci. USA 77, 2937 - 2940 (1980) entnommen werden.

Die antiarthritische Wirkung der erfindungsgemäßen Verbindungen kann in üblicher Weise im Tierexperiment im Adjuvans-Arthritis- oder Streptokokkenzellwandinduzierten-Arthritis-Modell bestimmt werden. Die dermatologische Aktivität, beispielsweise für die Behandlung von Akne, kann u.a. durch die komedolytische Aktivität und die Fähigkeit nachgewiesen werden, die Anzahl der Zysten im Modell der Rhino-Maus zu reduzieren.

Diese Methode ist von L.H. Kligman et al in The Journal of Investigative Dermatology 73, 354 - 358 (1978) beschrieben.

Dementsprechend sind ein weiterer Gegenstand der Erfindung therapeutische Mittel zur topischen und systemischen Anwendung, die eine Verbindung der Formel (I) neben üblichen Trägerstoffen oder Verdünnungsmitteln als Wirkstoff enthalten, und die Verwendung einer Verbindung der Formel (I) zur Herstellung eines Arzneimittels.

Die Herstellung der therapeutischen Mittel oder Zubereitungen erfolgt mit den üblichen flüssigen oder festen Trägerstoffen oder Verdünnungsmitteln und den in üblicher Weise verwendeten pharmazeutisch-technischen Hilfsstoffen, entsprechend der gewünschten Applikationsart und mit einer zur Anwendung geeigneten Dosierung, in üblicher Weise beispielsweise durch Vermischen des Wirkstoffes mit den an sich in solchen Präparaten üblichen festen und flüssigen Träger- und Hilfsstoffen.

Die Mittel können dementsprechend peroral, parenteral oder topisch verabreicht werden. Derartige Zubereitungen sind beispielsweise Tabletten, Filmtabletten, Dragees, Kapseln, Pillen, Pulver, Lösungen oder Suspensionen, Infusions- oder Injektionslösungen sowie Pasten, Salben, Gele, Cremes, Lotionen, Puder, Lösungen oder Emulsionen und Sprays.

Die therapeutischen Mittel können die erfindungsgemäß zu verwendenden Verbindungen bei lokaler Anwendung in 0,0001 bis 1 %iger Konzentration, bevorzugt in 0,001 bis 0,1 %iger Konzentration und bei systemischer Anwendung vorzugsweise in einer Einzeldosis von 0,1 bis 50 mg pro kg Körpergewicht enthalten und täglich in einer oder mehreren Dosierungen je nach Art und Schwere der Erkrankungen verabreicht werden.

Üblicherweise verwendete pharmazeutische technische Hilfsstoffe sind beispielsweise für die lokale Anwendung Alkohole, wie Ethanol, Isopropanol, oxyethyliertes Ricinusöl oder oxyethyliertes hydriertes Ricinusöl, Polyacrylsäure, Glycerinmonostearat. Paraffinöl, Vaseline, Wollfett, Polyethylenglykol 400, Polyethylenglykol 400-Stearat sowie ethoxylierter Fettalkohol, für die systemische Anwendung Milchzucker, Propylenglykol und Ethanol, Stärke, Talk, Polyvinylpyrrolidon. Den Präparaten kann gegebenenfalls ein Antioxidationsmittel, beispielsweise Tocopherol sowie butyliertes Hyroxyanisol oder butyliertes Hydroxytoluol oder geschmacksverbessernde Zusätze, Stabilisierungsmittel, Emulgiermittel, Bleichmittel usw. zugesetzt werden. Voraussetzung ist, daß alle bei der Herstellung pharmazeutischer Zubereitungen verwendeten Stoffe toxikologisch unbedenklich und mit den verwendeten Wirkstoffen verträglich sind.

Einige der erfindungsgemäßen Verbindungen weisen ein acides Wasserstoffatom auf und können daher mit Basen in üblicher Weise in ein physiologisch verträgliches, gut wasserlösliches Salz übergeführt werden. Geeignete Salze sind beispielsweise Ammonium- und Alkalimetallsalzek, insbesondere des Natriums, Kaliums und Lithiums und Erdalkalimetallsalze, insbesondere des Calciums oder Magnesiums, sowie Salze mit geeigneten organischen Basen, wie mit nieder-Alkylaminen, z.B. Methylamin, Ethylamin oder Cylohexylamin, oder mit substituierten nieder-Alkylaminen, insbesondere hydroxysubstituierten Alkylaminen, wie Diethanolamin, Triethanolamin oder Tris(hydroxymethyl)aminomethan, sowie mit Piperidin oder Morpholin.

Herstellung der erfindungsgemäßen Verbindungen

Beispiel 1

(E)-4-[2-(5,6,7,8-tetrahydro-5,5,8,8-tetramethyl-2-naphthalenyl)-1-propenyl]-benzolphosphonsäurediethylester

In einer Destillationsapparatur mit Tropftrichter wurden 20 g (52 mmol) (E)-1-(4-Bromphenyl)-2-(5,6,7,8-tetrahydro-5,5,8,8-tetramethyl-2-naphthalenyl)-1-propen und 0,86 g (7,5 mol%) wasserfreies Nickel(II)bromid unter Stickstoff auf ca. 160 °C erhitzt. 104 g (60 mmol) Triethylphoshit wurden daraufhin so zugetropft, daß die Zutropfgeschwindigkeit der Abdestillationsgeschwindigkeit des Ethylbromids entsprach. Man ließ 0,5 h nachrühren, dann abkühlen, versetzte mit wenig Toluol und engte ein. Der Rückstand wurde mit Essigester über Kieselgel filtriert. Man erhielt so 14,2 g (62 %) der Titelverbindung, Schmp. 72,5 -73,5 °C (aus Heptan).

Herstellung von 1-(Bromphenyl)-2-(5,6,7,8-Tetrahydro-5,5,8,8-tetramethyl-2-naphthalenyl)-1-propen

Zu einer Suspension von 2,4 g (0,1 mol) Natriumhydrid in 100 ml trockenem Dimethylsulfoxid wurde eine Lösung von 30,7 g (0,1 mol) 4-Brombenzylphosphonsäurediethylester in 50 ml Dimethylsulfoxid bei Raumtemperatur zugetropft. Man ließ 1 h nachrühren und tropfte dann eine Lösung von 17,3 g (0,075 mol) 2-Acetyl-5,6,7,8-tetrahydro-5,5,8,8-tetramethylnaphthalin in 25 ml Dimethylsulfoxid zu. Man ließ 16 h bei Raumtemperatur rühren, goß auf 1 l Wasser und saugte den entstandenen Niederschlag ab. Dieser wurde mit Wasser und anschließend Methanol gewaschen und nach Trocknen erhielt man 5,7 g 1-(Bromphenyl)-2-(5,6,7,8-Tetrahydro-5,5,8,8-tetramethyl-2-naphthalenyl)-1-propen. Schmp. 116 - 117 °C.

Beispiele 2 - 6

Analog Beispiel 1 wurden die Verbindungen der folgenden Tabelle 1 hergestellt.

Tabelle 1

| Bsp. | Name | A | $R^1$ | $R^2$ | $R^3$ | B | $R^4$ | $R^5$ | Fp (°C) |
|---|---|---|---|---|---|---|---|---|---|
| 2 | (E)-4-[2-(5,6,7,8-Tetrahydro-5,5,8,8-tetramethyl-2-naphthalenyl)-1-propenyl]-benzolphosphonsäuredimethylester | $CH_2CH_2$ | $CH_3$ | $CH_3$ | H | $C(CH_3)=CH$ | $OCH_3$ | $OCH_3$ | 97 - 98 |
| 3 | (E)-4-[2-2(5,6,7,8-Tetrahydro-5,5,8,8-tetramethyl-2-napthalenyl)-1-propenyl]-benzolphosphonsäure-dibutylester | $CH_2CH_2$ | $CH_3$ | $CH_3$ | H | $C(CH_3)=CH$ | $O$-n-$C_4H_9$ | $O$-n-$C_4H_9$ | 72 - 73 |
| 4 | (E)-4-[2-(2,3-Dihydro-1,1,2,3,3-pentamethyl-5(1H)-indenyl)-1-propenyl]benzolphosphonsäurediethylester | $CH(CH_3)$ | $CH_3$ | $CH_3$ | H | $C(CH_3)=CH$ | $OC_2H_5$ | $OC_2H_5$ | 84 - 85 |
| 5 | (E)-4-[2-(5,6,7,8-Tetrahydro-5,5,8,8-tetramethyl-2-naphthalenyl)-1-propenyl]-phenyl-phenylphosphinsäureethylester | $CH_2CH_2$ | $CH_3$ | $CH_3$ | H | $C(CH_3)=CH$ | $OC_2H_5$ | $C_6H_5$ | 101,5-103,5 |
| 6 | (E)-4-[2-(5,6,7,8-Tetrahydro-5,5,8,8-tetramethyl-2-naphthalenyl-1-propenyl]-phenyl-methylphosphinsäure-ethylester | $CH_2CH_2$ | $CH_3$ | $CH_3$ | H | $C(CH_3)=CH$ | $OC_2H_5$ | $CH_3$ | 119 - 120 |

Beispiel 7

4-[2-(5,6,7,8-Tetrahydro-5,5,8,8-tetramethyl-2-naphthalenyl)-ethinyl]-benzolphosphonsäurediethylester

4 g (19 mmol) 2-Ethinyl-5,6,7,8-tetrahydro-5,5,8,8-tetramethylnaphthalin, 5 g (17 mmol) 4-Brombenzolphosphonsäurediethylester, 17 ml Triethylamin, 2,6 mg Bis-(triphenylphosphin)-palladium-(II)-chlorid, 8,5 mg Kupfer-(I)-jodid und 17 mg Triphenylphosphin wurden in 5 ml Dimethylformamid 50 min unter Rückfluß erhitzt. Danach ließ man abkühlen, goß auf Wasser und extrahierte mit Ether. Die Etherphase wurde mit Wasser gewaschen, getrocknet ($Na_2SO_4$) und eingeengt. Der Rückstand wurde zweimal aus Heptan umkristallisiert und man erhielt so 2,6 g (33 %) der Titelverbindung, Schmp. 93 - 95 °C.

Beispiel 8

(E)-4-[2-(5,6,7,8,-Tetrahydro-5,5,8,8-tetramethyl-2-naphthalen-yl)-1-ethenyl]-benzolphosphonsäurediethylester

Zu einer Suspension von 1,8 g (64 mmol) Natriumhydrid in 50 ml trockenem Dimethylsulfoxid wurde eine Lösung von 22 g (53 mmol) 4-Diethylphosphonomethylbenzolphosphonsäurediethylester in 100 ml Dimethylsulfoxid bei Raumtemperatur zugetropft. Man ließ 1 h nachrühren und tropfte dann eine Lösung von 8,7 g (40 mmol) 2-Formyl-5,6,7,8-tetrahydro-5,5,8,8-tetramethyl-naphthalin in 75 ml Dimethylsulfoxid zu. Man ließ 16 h bei Raumtemperatur rühren, goß auf Wasser, säuerte mit 2 N Salzsäure an und extrahierte 3 x mit Ether. Die vereinigten organischen Phasen wurden 2 x mit Wasser und 2 x mit ges. Natriumchloridlösung gewaschen, getrocknet (Natriumsulfat) und eingeengt. Nach säulenchromatographischer Reinigung des Rückstands (Kieselgel; Heptan/0,55 - 10 % Essigester) erhielt man 5,5 g der Titelverbindung, Schmp. 83 - 84 °C.

Beispiel 9

(E)-4-[2-(5,6,7,8-Tetrahydro-5,5,8,8-tetramethyl-2-naphthalenyl)-1-propenyl]-benzolphosphonsäure

2 g (4,5 mmol) (E)-4-[2,-(5,6,7,8-Tetrahydro-5,5,8,8-tetramethyl-2-naphthalenyl)-1-propenyl]-benzolphosphonsäurediethylester, 1,86 g (18 mmol) Natriumbromid und 3 g (27 mmol) Trimethylchlorsilan wurden in 10 ml Acetonitril 3 h bei 40 °C gerührt. Danach ließ man abkühlen, filtrierte und engte das Filtrat ein. Das verbliebene Öl wurde 30 min. bei Raumtemperatur mit Wasser verrührt, dann extrahierte man mit Ether, wusch die organische Phase mit Wasser, trocknete (Natriumsulfat) und engte ein. Nach Umkristallisation des Rückstandes aus Essigsäure erhielt man 1,1 g der Titelverbindung, Schmp. 208 - 209 °C.

Beispiel 10

(E)-4-[2-(5,6,7,8-Tetrahydro-5,5,8,8-tetramethyl-2-naphthalenyl)-1-propenyl]-benzolphosphonsäuremonoethylester

1 g (2,3 mmol) (E)-4-[2-(5,6,7,8-Tetrahydro-5,5,8,8-tetramethyl-2-naphthalenyl)-1-propenyl]-benzolphosphonsäurediethylester wurde in ein Gemisch aus 8,9 ml Ethanol und 5,9 ml 10 N NaOH 30 min. unter Rückfluß erhitzt. Nach dem Abkühlen wurde der entstandene Niederschlag abgesaugt und getrocknet. Dann wurde er in Wasser wieder gelöst und mit 2 N HCl angesäuert. Absaugen und Trocknen des Niederschlags ergab 0,6 g der Titelverbindung, Schmp. 165 - 166 °C.

**Patentansprüche**

1. Arylphosphorderivate der allgemeinen Formel

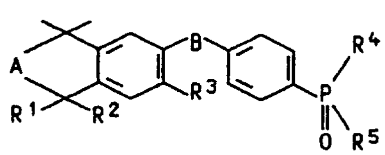

I,

in der
A einen gegebenenfalls mit einer Methylgruppe substituierten Ethylen- oder Methylenrest,
B eine $-CR^6=CH-$ oder $-C\equiv C-$Gruppe,
$R^1$ und $R^2$ Wasserstoff oder einen Methylrest,
$R^3$ Wasserstoff, einen $C_{1-4}$-Alkylrest, ein Halogenatom, vorzugsweise Fluor, oder eine Methoxygruppe und
$R^4$ und $R^5$ einen $C_{1-6}$-Alkylrest, einen Phenylrest, einen Rest $-OR^7$ oder einen Rest $-NR^8R^9$ bedeuten, wobei
$R^6$ für Wasserstoff oder einen $C_{1-4}$-Alkylrest,
$R^7$, $R^8$ und $R^9$ für Wasserstoff, einen gegebenenfalls durch eine Hydroxylgruppe substituierten $C_{1-4}$-Alkylrest oder eine Phenylgruppe stehen,
wobei zwei an Phosphor gebundene Heteroatome durch eine $C_{2-3}$-Alkylengruppe verbunden sein können, oder $R^8$ und $R^9$ zusammen mit dem Stickstoffatom, an das sie gebunden sind, für einen heterocyclischen Rest, vorzugsweise Morpholin, Pyrrolidin oder Piperidin, stehen, sowie deren physiologisch verträglichen Salze.

**2.** Arylphosphorderivate der allgemeinen Formel I nach Anspruch 1, dadurch gekennzeichnet, daß
$R^1$ und $R^2$ einen Methylrest,
$R^3$ Wasserstoff,
$R^4$ und $R^5$ unabhängig voneinander je einen $C_{1-6}$-Alkylrest, einen Phenylrest, Hydroxyl oder $C_{1-4}$-Alkoxy darstellen.

**3.** Verfahren zur Herstellung der Arylphosphorderivate der allgemeinen Formel I nach Anspruch 1 oder 2, dadurch gekennzeichnet, daß man
a) einen Aldehyd oder ein Keton der Formel II

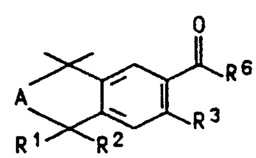

II,

in der $R^1$, $R^2$, $R^3$, $R^6$ und A die oben angegebenen Bedeutungen haben, in einer Wittig-Horner-Reaktion mit einem Phosphonat der Formel III

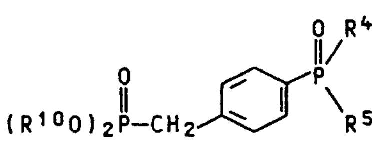

III,

in der $R^4$ und $R^5$ die oben angegebene Bedeutung haben und $R^{10}$ eine $C_{1-4}$-Alkylgruppe bedeutet, umsetzt oder
b) eine Phosphoniumverbindung der Formel IV

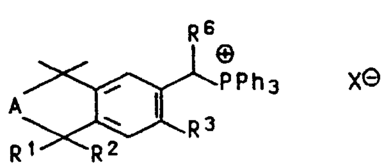

IV,

in der $R^1$, $R^2$, $R^3$, $R^6$ und A die oben angegebenen Bedeutungen haben und X ein anorganisches Anion ist, in einer Wittig-Reaktion mit einem Aldehyd der Formel V

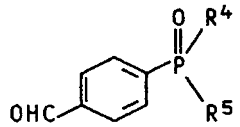

V,

in der $R^4$ und $R^5$ die oben angegebene Bedeutung haben, umsetzt oder
c) indem man ein Arylhalogenid der Formel VI

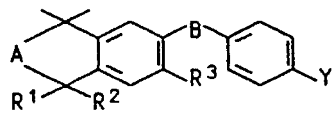

VI,

in der $R^1$, $R^2$, $R^3$, A und B die oben angegebenen Bedeutungen haben und Y ein Halogenatom bedeutet, zweckmäßigerweise in Gegenwart eines Übergangsmetallkatalysators, mit einer Phosphorverbindung der Formel VII

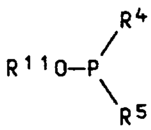

VII,

worin $R^4$ und $R^5$ die oben angegebene Bedeutung haben und $R^{11}$ einen $C_{1-6}$-Alkylrest bedeutet, umsetzt oder
d) ein Arylacetylen der Formel VIII

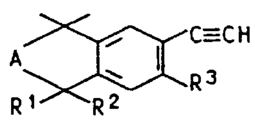

VIII,

worin $R^1$, $R^2$, $R^3$ und A die oben angegebenen Bedeutungen haben, mit einem Arylhalogenid der Formel IX

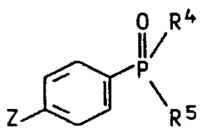

IX

worin $R^4$ und $R^5$ die obene angegebene Bedeutung haben und Y für ein Halogenatom steht, zweckmäßig in Gegenwart eines Übergangsmetallkatalysators umsetzt und gegebenenfalls
e) die mit den Verfahren a) - c) erhaltenen Verbindungen nach Standardmethoden in weitere Verbindungen der Formel I umwandelt.

4. Für die orale, parenterale oder topische Anwendung geeignete pharmazeutische Zubereitung, dadurch gekennzeichnet, daß sie neben den für die jeweilige Anwendungsart üblichen galenischen Hilfsstoffen als Wirkstoff ein Arylphosphorderivat der allgemeinen Formel I nach Anspruch 1 oder 2 oder ein physiologisch verträgliches Salz davon in wirksamer Menge enthält.

5. Verwendung eines Arylphosphorderivats der Formel I nach Anspruch 1 oder 2 oder eines physiologisch verträglichen Salzes davon zur Herstellung eines pharmazeutischen Mittels.

6. Verwendung eines Arylphosphorderivats der Formel I nach Anspruch 1 oder 2 oder eines physiologisch verträglichen Salzes davon zur Herstellung eines Mittels gegen Akne, Psoriasis und anderer, mit pathologisch veränderter Verhornung einhergehender dermatologischer Erkrankungen sowie zur Behandlung von rheumatischen Erkrankungen und zur prophylaktischen Behandlung von Praekanzerosen und Tumoren.

## Claims

1. An arylphosphorus derivative of the formula

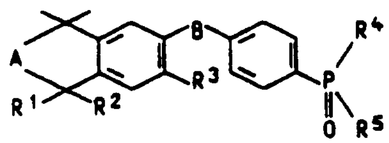

I

where A is an unsubstituted or methyl-substituted ethylene or methylene radical, B is a $-CR^6=CH-$ or $-C\equiv C-$group, $R^1$ and $R^2$ are each hydrogen or methyl, $R^3$ is hydrogen, $C_1$-$C_4$-alkyl, halogen, preferably fluorine, or methoxy and $R^4$ and $R^5$ are each $C_1$-$C_6$-alkyl, phenyl, a radical $-OR^7$ or $-NR^8R^9$, $R^6$ is hydrogen or $C_1$-$C_4$-alkyl, $R^7$, $R^8$ and $R^9$ are each hydrogen, an unsubstituted or hydroxyl-substituted $C_1$-$C_4$-alkyl radical or phenyl, where two hetero atoms bonded to phosphorus may be linked by a $C_2$- or $C_3$-alkylene group, or $R^8$ and $R^9$, together with the nitrogen atom to which they are bonded, form a heterocyclic radical, preferably a morpholine, pyrrolidine or piperidine radical, and its physiologically tolerated salts.

2. An arylphosphorus derivative of the formula I as claimed in claim 1, wherein $R^1$ and $R^2$ are each methyl, $R^3$ is hydrogen and $R^4$ and $R^5$ independently of one another are each $C_1$-$C_6$-alkyl, phenyl, hydroxyl or $C_1$-$C_4$-alkoxy.

3. A process for the preparation of an aryl-phosphorus derivative of the formula I as claimed in claim 1 or 2,
wherein
a) an aldehyde or a ketone of the formula II

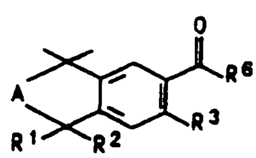

II

where $R^1$, $R^2$, $R^3$, $R^6$ and A have the abovementioned meanings, is subjected to a Wittig-Horner reaction with a phosphonate of the formula III

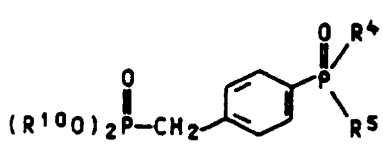

III

where $R^4$ and $R^5$ have the abovementioned meanings and $R^{10}$ is $C_1$-$C_4$-alkyl, or

b) a phosphonium compound of the formula IV

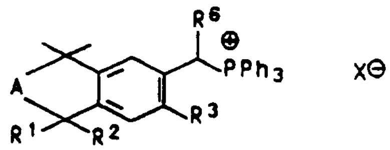

IV

where $R^1$, $R^2$, $R^3$, $R^6$ and A have the abovementioned meanings and X is an inorganic anion, is subjected to a Wittig reaction with an aldehyde of the formula V

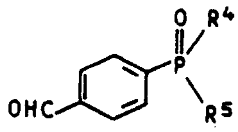

V

where $R^4$ and $R^5$ have the abovementioned meanings, or
c) an aryl halide of the formula VI

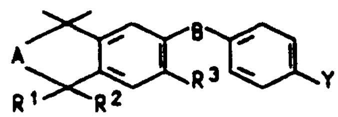

VI

where $R^1$, $R^2$, $R^3$, A and B have the abovementioned meanings and Y is halogen, is reacted with a phosphorus compound of the formula VII

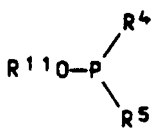

VII

where $R^4$ and $R^5$ have the abovementioned meanings and $R^{11}$ is $C_1$-$C_6$-alkyl, advantageously in the presence of a transition metal catalyst, or
d) an arylacetylene of the formula VIII

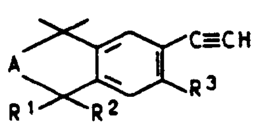

VIII

where $R^1$, $R^2$, $R^3$ and A have the abovementioned meanings, is reacted with an aryl halide of the formula IX

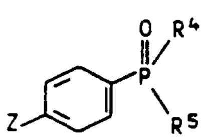

IX

where $R^4$ and $R^5$ have the abovementioned meanings and Y is halogen, advantageously in the presence of a transition metal catalyst, and, if required,
e) the compound obtained in processes a) - c) is converted into another compound of the formula I by a standard method.

4. A pharmaceutical formulation suitable for oral, parenteral or topical use, which contains an arylphosphorus derivative of the formula I as claimed in claim 1 or 2 or a physiologically tolerated salt thereof as the active compound in an effective amount, in addition to the pharmaceutical auxiliaries usually employed for the particular route of administration.

5. Use of an arylphosphorus derivative of the formula I as claimed in claim 1 or 2 or of a physiologically tolerated salt thereof for the preparation of a pharmaceutical agent.

6. Use of an arylphosphorus derivative of the formula I as claimed in claim 1 or 2 or of a physiologically tolerated salt thereof for the preparation of an agent against acne, psoriasis and other dermatological diseases accompanied by pathologically changed cornification and for the treatment of rheumatic disorders and for the prophylactic treatment of precancerous stages and tumors.

**Revendications**

1. Dérivés arylphosphorés de la formule générale

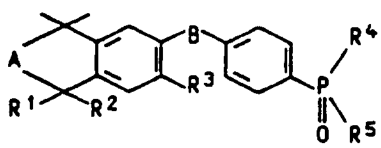

I,

dans laquelle
A représente un radical méthylène ou éthylène éventuellement substitué par un radical méthyle,
B représente un radical $-CR^6=CH-$ ou $-C\equiv C-$,
$R^1$ et $R^2$ représentent des atomes d'hydrogène ou des radicaux méthyle,
$R^3$ représente un atome d'hydrogène, un radical alkyle en $C_1$-$C_4$, un atome d'halogène, de préférence de fluor, ou un radical méthoxy et
$R^4$ et $R^5$ représentent chacun un radical alkyle en $C_1$-$C_6$, un radical phényle, un radical $-OR^7$ ou un radical $-NR^8R^9$, où
$R^6$ représente un atome d'hydrogène ou un radical alkyle en $C_1$-$C_4$,
$R^7$, $R^8$ et $R^9$ représentent chacun un atome d'hydrogène, un radical phényle, ou un radical alkyle en $C_1$ à $C_4$, éventuellement substitué par un radical hydroxyle,
où deux hétéroatomes liés au phosphore peuvent être liés par un radical alkylène en $C_2$-$C_3$, ou bien $R^8$ et $R^9$ représentent, ensemble avec l'atome d'azote auquel ils sont attachés, un radical hétérocyclique, de préférence un radical morpholinyle, pyrrolidinyle ou pipéridinyle, ainsi que leurs sels physiologiquement compatibles.

2. Dérivés arylphosphorés de la formule générale I suivant la revendication 1, caractérisés en ce que
$R^1$ et $R^2$ représentent chacun un radical méthyle,
$R^3$ représente un atome d'hydrogène,
$R^4$ et $R^5$ représentent, chacun indépendamment l'un de l'autre, un radical alkyle en $C_1$-$C_6$, un radical phényle, un radical hydroxyle ou un radical alcoxy en $C_1$-$C_4$.

3. Procédé de préparation des dérivés arylphosphorés de la formule générale I selon la revendication 1 ou 2, caractérisé en ce que

a) on fait réagir un aldéhyde ou une cétone de la formule II

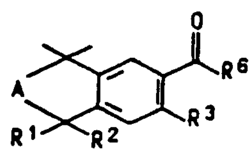

II.

dans laquelle $R^1$, $R^2$, $R^3$, $R^6$ et A possèdent les significations qui leur ont été attribuées ci-dessus, selon une réaction de Wittig-Horner, avec un phosphonate de la formule III

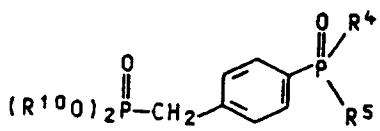

III.

dans laquelle $R^4$ et $R^5$ possèdent les significations qui leur ont été attribuées ci-dessus et $R^{10}$ représente un radical alkyle en $C_1$-$C_4$, ou bien

b) on fait réagir un composé de phosphonium de la formule IV

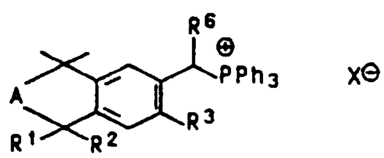

IV.

dans laquelle $R^1$, $R^2$, $R^3$, $R^6$ et A possèdent les significations qui leur ont été attribuées ci-dessus et X représente un anion inorganique, selon une réaction de Wittig, avec un aldéhyde de la formule V

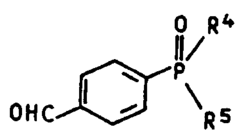

V.

dans laquelle $R^4$ et $R^5$ possèdent les significations qui leur ont été attribuées ci-dessus, ou bien

c) on fait réagir un halogénure d'aryle de la formule VI

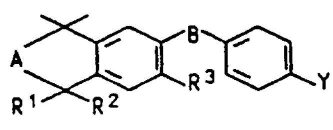

VI.

dans laquelle $R^1$, $R^2$, $R^3$ A et B possèdent les significations qui leur ont été attribuées ci-dessus et Y représente un atome d'halogène, avantageusement en présence d'un catalyseur à métal de transition, avec un composé du phosphore de la formule VII

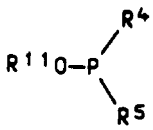

VII.

dans laquelle $R^4$ et $R^5$ possèdent les significations qui leur ont été attribuées ci-dessus et $R^{11}$ représente un radical alkyle en $C_1$-$C_6$, ou bien

d) on fait réagir un arylacétylène de la formule VIII

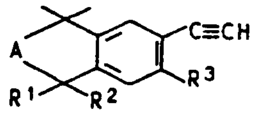

VIII.

dans laquelle $R^1$, $R^2$, $R^3$ et A possèdent les significations qui leur ont été attribuées ci-dessus, avec un halogénure d'aryle de la formule IX

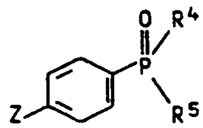

IX

dans laquelle $R^4$ et $R^5$ possèdent les significations qui leur ont été attribuées ci-dessus et Y représente un atome d'halogène, avantageusement en présence d'un catalyseur à métal de transition et, éventuellement,

e) on convertit les composés obtenus selon les procédés a)-c) en d'autres composés de la formule I suivant des méthodes standardisées.

**4.** Préparation pharmaceutique convenant à l'administration par la voie orale, parentérale ou topique, caractérisée en ce qu'elle contient, outre les excipients ou adjuvants galéniques habituels pour le domaine d'utilisation envisagé, à titre de principe actif, un dérivé arylphosphoré de la formule I suivant la revendication 1 ou 2, ou un sel physiologiquement compatible de ce dérivé, en une proportion efficace.

**5.** Utilisation d'un dérivé arylphosphoré de la formule I selon la revendication 1 ou 2, ou d'un sel physiologiquement compatible de ce dérivé en vue de la préparation d'un agent ou d'une composition pharmaceutique.

**6.** Utilisation d'un dérivé arylphosphoré de la formule I selon la revendication 1 ou 2, ou d'un sel physiologiquement compatible de ce dérivé, en vue de la préparation d'un agent ou d'une composition pharmaceutique contre l'acné, le psoriasis et d'autres maladies dermatologiques s'accompagnant d'une kératinisation pathologiquement modifiée, comme aussi en vue du traitement de maladies rhumatismales et en vue du traitement prophylactique d'états précancéreux et tumoraux.